# EUROPÄISCHE PATENTSCHRIFT

(11) **EP 2 677 977 B1**
(45) Veröffentlichungstag und Bekanntmachung des Hinweises auf die Patenterteilung: **08.04.2015**
(21) Anmeldenummer: 12708272.5
(22) Anmeldetag: 22.02.2012
(51) Int. Cl.: A61F 5/01

(54) **RÜCKEN- ODER BECKENBANDAGE**
BACK OR PELVIC BELT
BANDAGE POUR LE DOS OU LE BASSIN

(30) Priorität: 25.02.2011 DE 102011000953
(43) Veröffentlichungstag der Anmeldung: 01.01.2014
(73) Patentinhaber: Bauerfeind AG, 07937 Zeulenroda-Triebes (DE)
(72) Erfinder: BAUERFEIND, Hans, 07937 Zeulenroda (DE)
(74) Vertreter: Puschmann Borchert Bardehle Patentanwälte Partnerschaft mbB
(86) Internationale Anmeldenummer: PCT/EP2012/052999
(87) Internationale Veröffentlichungsnummer: WO 2012/113823

(56) Entgegenhaltungen:
- US-A- 3 926 183
- US-A- 4 794 916
- US-A- 5 551 085

## Beschreibung

Die Erfindung bezieht sich auf eine Rücken- oder Beckenbandage mit zwei benachbarten Pelotten, die auf einem Tragstück im Rückenbereich der Bandage angebracht sind, an dessen gegenüberliegenden Enden jeweils ein Schließbandstück zum Anlegen der Bandage durch Zusammenfassen der Enden der beiden Schließbandstücke ansetzen.

Eine derartige Bandage ist in der deutschen Offenlegungsschrift 23 34 500 offenbart. Die bekannte Bandage dient dazu, Erkrankungen der Wirbelsäule zu behandeln und entsprechende Schmerzen zu lindern, wozu im Wesentlichen die Pelotten dienen, die zu beiden Seiten der Wirbelsäule dauernd auf das Muskelgewebe drücken, so dass die Rückenmuskulatur durch einen von hinten nach vorn gerichteten Druck ständig massiert und gestärkt wird. Die Pelotten werden von einem Teil eines um den Leib spannbaren Gurts gehalten und bleiben in ihrer Position unverändert.

Eine gattungsgemäße, sämtliche Merkmale des Oberbegriffs des Patentanspruches 1 aufweisende Bandage ist in der US 4,794,916 offenbart.

Der Erfindung liegt die Aufgabe zugrunde die bekannte Bandage dahingehend zu verbessern, dass die Schmerzlinderung intensiviert wird. Erfindungsgemäß geschieht dies dadurch, dass jeder Zuggurt jeweils zu einer Zugöse führt und in dieser endet, durch die jeweils ein Spannband geführt ist, das mit seinem einen Ende zu einem Schließbandstück und seinem anderen Ende frei zum Erfassen derart geführt ist, dass bei Spannung der beiden Spannbänder das Tragstück sich zusammenzieht und die Pelotten einander annähern. Die freien Enden der beiden Spannbänder lassen sich auf diese Weise von Hand ziehen, womit die Spannbänder über die

Umlenkösen einen Zug ausüben, der sich auf die Zuggurte fortsetzt und damit eine gegenläufige Spannung auf das Tragstück ausübt, durch die das Tragstück zusammengezogen wird und damit die Pelotten einander annähern.

Hierdurch ergibt sich, dass auf sogenannte "Triggerpoints" in der Gesäßmuskulatur ein Massageeffekt ausgeübt wird und sich diese damit entspannt. Die Entspannung beeinflusst die Positionierung der Gelenkflächen der Kreuzdarmbeingelenke günstig. Die sich einander annähernden Pelotten erzwingen eine Hebelwirkung- ähnlich einer Zangenwirkung - auf dem hinteren vorstehenden Teil des Darmbeins, wodurch ein Lockerungseffekt auf die im inneren Beckenbereich liegenden Kreuzdarmbeingelenke entsteht. Insbesondere beim Gehen tritt dieser Effekt durch die wechselseitige Anspannung der Gesäßmuskeln auf.

Durch die besonderen Zuggurte und deren Führung ergibt sich der Effekt einer räumlichen Zusammenziehung der benachbarten Pelotten, die damit nicht nur einen Druck nach innen in den Körper ausüben, sondern insbesondere eine Schubwirkung von der Seite auf die Wirbelsäule erzeugen, die eine hebelarmähnliche Wirkung auslöst, so dass im Bereich der Kreuzdarmbeingelenke ein Repositionierungseffekt bei fehlerhafter Stellung derselben (Blockierung) ausgelöst wird.

Nach Beseitigung der fehlerhaften Stellung stabilisieren jetzt die Zuggurte die Kreuzdarmbeingelenke und den gesamten Beckengürtel inklusive der Symphyse in günstiger Position und sichern damit dauerhaft den eingetretenen schmerzlindernden Effekt.

Um an der Rücken- bzw. Beckenbandage unterschiedliche Pelotten anbringen zu können, wird das Tragstück mit einer Klettverschlussoberfläche versehen, die zu den entsprechenden Klettverschlussoberflächen der Pelotten passt, so dass verschiedene Pelotten über die Klettverschlüsse in einfacher Weise ausgetauscht werden können.

In den Figuren ist ein Ausführungsbeispiel der Erfindung dargestellt. Es zeigen
- Figur 1: die Bandage mit geschlossenen Schließbandstücken und offenen Spannbändern mit Blickrichtung auf die Innenseite des Tragstücks;
- Figur 2: die Bandage in der gleichen Lage wie in Figur 1 dargestellt, jedoch mit Blickrichtung auf die Innenseite der geschlossenen Schließbandstücke;

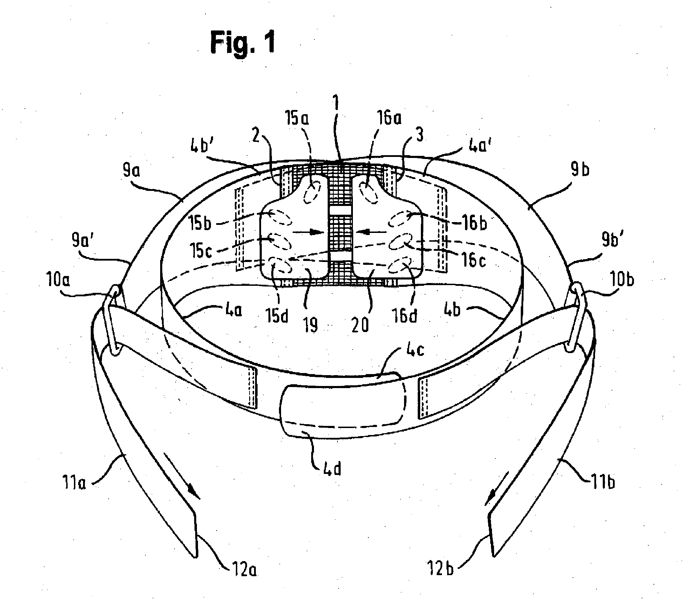

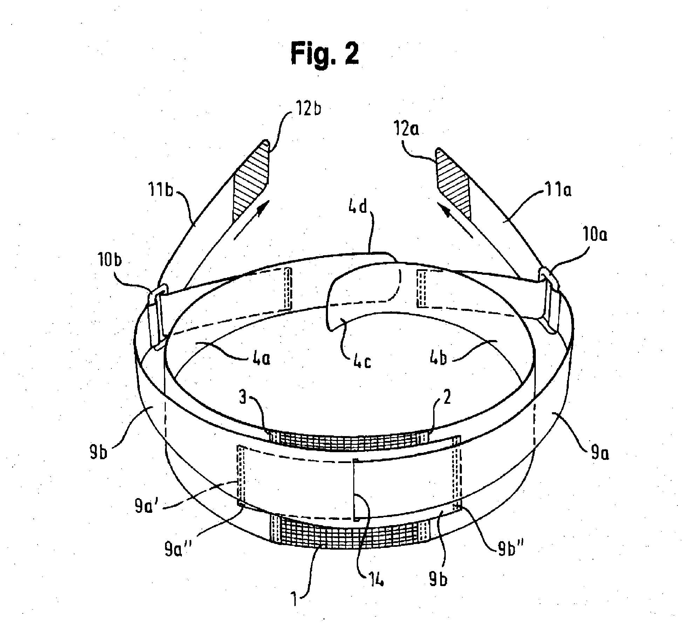

- Fig. 1 und 2: spiegeln nicht die Erfindung gemäß Anspruch 1 mit einem Umlenkstück wieder.

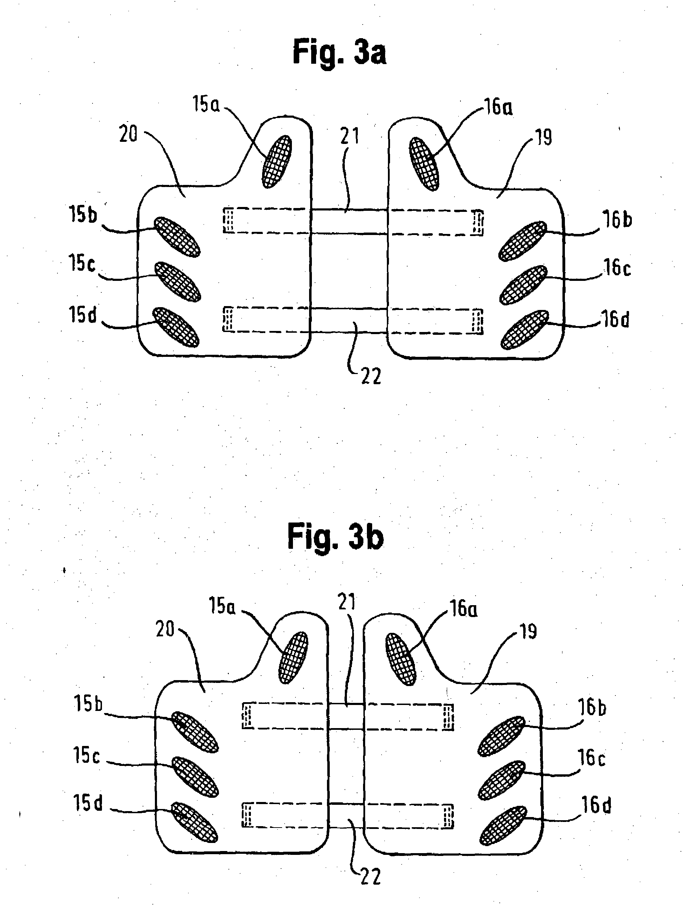

- Figuren 3a und 3b: das Tragstück mit auseinander gezogenen und miteinander angenäherten Pelotten;

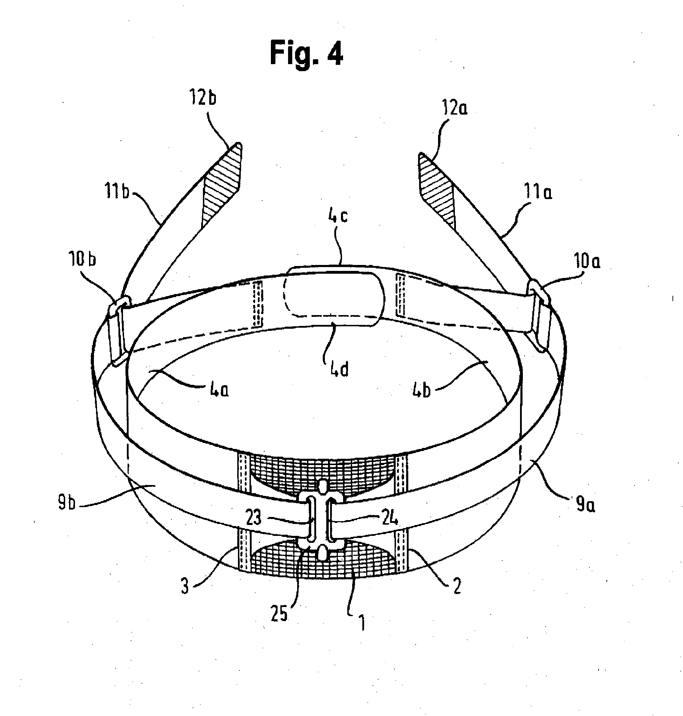

- Figur 4: die Bandage in einer Lage wie in Figur 2 dargestellt, allerdings mit einem erfindungsgemäßen Umlenkstück zur gegenläufigen Führung der Zuggurte;

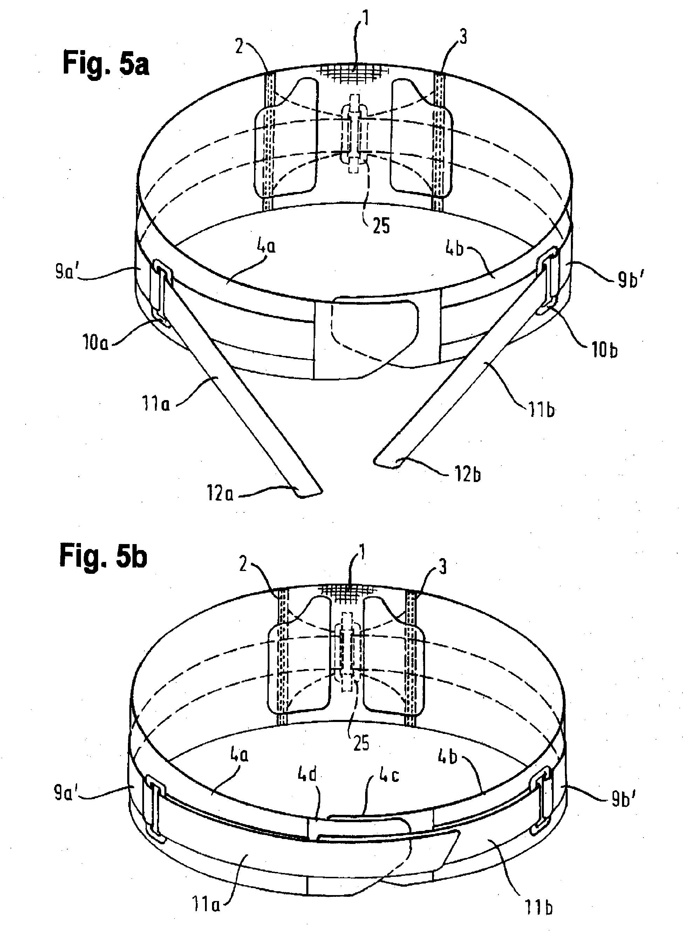

- Figuren 5a und 5b: die Bandage in einer Lage wie in Figur 1 dargestellt, allerdings mit einem Umlenkstück am Tragstück und zwar in Figur 5a in entspannter Lage des Tragstücks und Figur 5b in zusammengezogener Lage des Tragstücks;

Figur 1 zeigt die Bandage mit Blick auf die Innenseite des elastischen Tragstücks 1 mit acht daran angebrachten Pelotten 15a, 15b, 15c, 15d und 16a,16b, 16c, 16d. An den beiden Seiten 2 und 3 des Tragstücks 1 sind die Enden 4a' und 4b' der beiden Schließbandstücke 4a und 4b befestigt. Diese dienen dazu, den Körper des Patienten zu umschlingen und mit ihrem Zusammenziehen das elastische Tragstück 1 zu dehnen. In der dargestellten Umschlingungslage werden die Schließbandstücke 4a und 4b an ihren beiden Enden 4c und 4d durch Überlappung zu einem geschlossenen Ring verbunden, wobei die Enden 4c und 4d als Klettverschlüsse ausgebildet sind. Damit besteht die Möglichkeit, die Schließbandstücke 4a und 4b jederzeit zum Abnehmen der Bandage zu öffnen und nach ihrer Anlage am Körper des Patienten wieder zu schließen.

Auf der dem Betrachter abgewandten Seite des Tragstücks 1 sind die Enden 9a' und 9b' der beiden Zuggurte 9a und 9b befestigt. Die beiden Zuggurte 9a und 9b umfassen teilweise die Schließbandstücke 4a und 4a und enden in den beiden Ösen 10a und 10b, durch die jeweils ein Spannband 11a und 11 b hindurchgezogen ist. Mit den Spannbändern 11 a und 11b wird bei deren Anziehen an ihren freien Enden 12a und 12b in der eingezeichneten Pfeilrichtung ein Zug auf die Zuggurte 9a und 9b nach Art eines Flaschenzuges ausgeübt, durch den wegen einer anhand der Figur 2 näher erläuterten gegenläufigen Führung der beiden Zuggurte 9a und 9b über das elastische Tragstück 1 dieses sich zusammenzieht bzw. bei Lockerung der Zuggurte 9a und 9b entspannt. Nach erfolgtem Anlegen der beiden Schließbandstücke 4a und 4b werden diese mit ihren Enden 4c und 4d, die als Klettverschlüsse ausgebildet sind, miteinander verbunden, so dass damit die Bandage mit mehr oder weniger gedehntem Tragstück 1 und in gewünschter Lage der Pelotten 14, 15, 17 und 18 fest am Körper ihren erforderlichen Sitz erhalten hat.

Figur 2 zeigt die Bandage gemäß Figur 1 in einer Lage mit Blickrichtung auf die gegenläufige Führung der Zugurte 9a und 9b über dem Tragstück 1. In der Figur 2 ist weiter die besondere Befestigung der betreffenden Enden 9a' und 9b' der Zuggurte 9a und 9b an den Schließbandstücken 4a und 4b dargestellt. Der Zuggurt 9a umfasst das Tragstück 1 und ist mit seinem Ende 9a' über die Naht 9a" mit dem Schließbandstück 4a fest verbunden. Der Zuggurt 9b liegt zunächst auf dem in der Naht 9a" befestigten Ende 9a' des Zuggurtes 9a auf und enthält den Schlitz 14 im Zuggurt 9b und schiebt sich neben den Schlitz 14 und schließlich zum Ende 9b' mit der Naht 9b" und ist somit fest mit dem Schließbandstück 4b verbunden. Wie ersichtlich, ist die Naht 9b" von dem Zuggurt 9a teilweise überdeckt. Diese Gestaltung der in den Nähten 9a" und 9b" endenden Zuggurte 9a und 9b führt bei Anziehen der Spannbänder 11 a und 11b zu einem über die Ösen 10a und 10b geleiteten, gegenläufigen Zug auf die Zuggurte 9a und 9b, durch die die an den Nähten 9a" und 9b"festgehaltenen Enden 9a' und 9b' der Zuggurte 9a und 9b zusammengezogen werden. Dieser Zug führt zu einer Annäherung der Nähte 9a" und 9b" an dem Tragstück 1, womit auf die Rückenbandage ein das Tragstück 1 zusammenziehende Kraft und damit die Verengung der Rückenbandage verursacht wird.

In der Figur 3 a sind die Pelotten 15a, 15b, 15c, 15d und 16a,16b, 16c, 16d auf ihren Unterlagen 19 und 20 in der Lage dargestellt, wie sie auch in Figur 1 wiedergegeben ist, das heißt in auseinander gezogener Lage. Die beiden Träger 19 und 20 werden dabei durch die elastischen Bänder 21 und 22 miteinander verbunden.

Figur 3b zeigt die beiden Träger 19 und 20 mit den vier Pelotten 15a, 15b, 15c, 15d und 16a,16b, 16c, 16d in zusammengezogener Lage, wie sie sich ergibt, wenn das dazugehörige Tragstück 1 zusammengezogen ist (siehe Figur 5b), sofern die beiden Spannbänder 11 a und 11 b entsprechend gespannt sind.

In der Figur 4 ist eine Bandage dargestellt, die bis auf die Gestaltung am Tragstück 1 mit derjenigen gemäß Figur 2 übereinstimmt, so dass diesbezüglich auf die Erläuterungen zu Figur 2 verwiesen werden kann. Bei der Ausführungsform gemäß Figur 4 werden die beiden Zuggurte 9a und 9b jeweils durch Schlitze 23 und 24 unter Richtungsumkehr durch das Umlenkstück 25 geführt und dann jeweils an den betreffenden Seiten 2 und 3 des Tragstücks 1 fest angebracht. Bei Spannung der beiden Zuggurte 9a und 9b ergibt sich aufgrund dieser Richtungsumkehr eine Zusammenziehung des Tragstücks 1 und damit eine entsprechende Annäherung der daran angebrachten Pelotten, wie dies in der Figur 3b dargestellt ist.

In den Figuren 5a und 5b ist die Gestaltung der Bandage gemäß Figur 4 in einer Lage dargestellt, in der der Blick auf die Innenseite des Tragstücks 1 gerichtet ist, wobei in Figur 5a das Tragstück 1 gedehnt und in Figur 5b zusammengezogen dargestellt ist, wie sich die letztere Lage aufgrund der Spannung der Zuggurte 9a und 9b ergibt. Hinsichtlich der anderen Bestandteile der Bandage wird auf die Darstellung in den Figuren 1 und 2 und deren Erläuterung verwiesen. Die Pelotten sind aus Gründen der Übersichtlichkeit nicht mitgezeichnet, da ihre Anordnung aus Fig. 1 deutlich hervorgeht.

Es sei noch darauf hingewiesen, dass in Figur 5b die Bandage in geschlossener Lage dargestellt ist, in der die beiden Schließbandstücke 4a und 4b geschlossen gezeichnet sind, wobei über der Schließlage, wie in Figur 1 dargestellt, auch die beiden Zugurte 11 a und 11 b in ihrer Schließlage gezeichnet sind, wodurch die Bandage insgesamt in sich geschlossen ist.

## Patentansprüche

1. Rücken- oder Beckenbandage mit benachbarten Pelotten (15, 16, 17, 18), die auf einem Tragstück (1) im Rückenbereich der Bandage angebracht sind, an dessen gegenüberliegenden Randbereichen (2, 3) jeweils ein Schließbandstück (4a, 4b) zum Anlegen der Bandage durch Zusammenfassen der Enden der beiden Schließbandstücke (4a, 4b) ansetzt, wobei dem Tragstück (1) zwei Zuggurte (9a, 9b) zugeordnet sind, die derart geführt sind, dass die Pelotten (15, 16, 17, 18) bei Spannung beider Zuggurte (9a, 9b) sich annähern und auf das betreffende Körperteil einen von einer auf die Zuggurte (9a, 9b) wirkenden Zugkraft abhängigen Druck ausüben, **dadurch gekennzeichnet,** dass die Zuggurte (9a, 9b) je über ein zwischen den Pelotten (15, 16, 17, 18) angebrachtes Umlenkstück (25) unter Richtungsumkehr zu dem jeweiligen Randbereich (2, 3) des Tragstücks (1) geführt sind.

2. Rücken- oder Beckenbandage nach Anspruch 1, **dadurch gekennzeichnet,** dass jeder Zuggurt (9a, 9b) jeweils zu einer Zugöse (10a, 10b) führt und in dieser endet, durch die jeweils ein Spannband (11a, 11b) geführt ist, das mit seinem einen Ende zu einem Schließbandstück (4a, 4b) und seinem anderen Ende frei zum Erfassen derart geführt ist, dass bei Spannung der beiden Spannbänder (11a, 11b) das Tragstück (1) sich zusammenzieht und die Pelotten (15, 16, 17, 18) einander annähern.

3. Rückenbandage nach Anspruch 1 oder 2, **dadurch gekennzeichnet,** dass das Tragstück (1) mit einer Klettverschlussoberfläche passend zu den mit entsprechenden Klettverschlussoberflächen gestalteten Pelotten (15, 16, 17, 18) versehen ist.

## Claims

1. Back or pelvic brace with adjacent pads (15, 16, 17, 18) which are attached to a support element (1) provided on the back portion of said brace, with a closure strap piece (4a, 4b) each extending from opposite edge areas (2, 3) of said support element (1) for applying said brace by bringing together the ends of said two closure strap pieces (4a, 4b), said support element (1) furthermore having two associated tensioning belts (9a, 9b) which are guided such that said pads (15, 16, 17, 18) will approach each other when the two tensioning belts (9a, 9b) are tightened, thus exerting a pressure on the respective body part, which pressure is a function of the tension acting on said tensioning belts (9a, 9b) **characterized** in that said tensioning belts (9a, 9b) are each guided to the respective edge area (2, 3) of said support element (1) via a diverting element (25) which is positioned between said pads (15, 16, 17, 18) and which acts to reverse the direction of the course of each belt.

2. Back or pelvic brace as claimed in claim 1 **characterized in** that each tensioning belt (9a, 9b) extends to and terminates in a towing eye (10a, 10b) through which a tightening strap (11a, 11 b) is guided, one end of which strap extends to a closure strap piece (4a, 4b) and the other end thereof can be gripped freely in such a way that tightening of said two tightening straps (11a, 11b) will result in the support element (1) being contracted, thus causing said pads (15, 16, 17, 18) to approach each other.

3. Back or pelvic brace as claimed in claims 1 or 2 **characterized in** that said support element (1) has a hook and loop fastener surface matching the hook and loop fastener surfaces provided on said pads (15, 16, 17, 18).

## Revendications

1. Bandage pour le dos ou le bassin avec des inserts adjacents (15, 16, 17, 18) montés sur une pièce porteuse (1) dans le dos du bandage dont les rebords opposés (2, 3) disposent chacun d'une portion de bande de fermeture (4a, 4b) servant à mettre le bandage en attachant ensemble les extrémités des deux portions de bande de fermeture (4a, 4b), la pièce porteuse (1) disposant quant à elle de deux sangles (9a, 9b) agencées de sorte que les inserts (15, 16, 17,18), lorsque les deux sangles (9a, 9b) sont tendues, se rapprochent l'une de l'autre et exercent sur la partie du corps concernée une pression dépendante de la force de traction exercée sur les sangles (9a, 9b), **caractérisé en** ce que les sangles (9a, 9b) passent toutes deux par une pièce de renvoi (25) située entre les inserts (15, 16, 17, 18) pour faire demi-tour jusqu'au rebord respectif (2, 3) de la pièce porteuse (1).

2. Bandage pour le dos ou le bassin selon la revendication 1, **caractérisé en** ce que chaque sangle (9a, 9b) se rend et termine dans un anneau d'attelage (10a, 10b) à travers lequel est introduite une bande de serrage (11, 11 b) dont l'une des extrémité mène à une portion de bande de fermeture (4a, 4b) et dont l'autre extrémité, qui peut être saisie, est agencée de façon à compresser la pièce porteuse (1) lorsque les deux bandes de serrage (11 a, 11 b) sont tendues, rapprochant ainsi les inserts (15, 16, 17, 18) entre eux.

3. Bandage pour le dos selon les revendications 1 ou 2, **caractérisés en** ce que la pièce porteuse (1) est munie d'une surface en Velcro qui s'agence avec les surfaces en Velcro correspondantes des inserts (15, 16, 17, 18).
